# EUROPEAN PATENT APPLICATION

(11) **EP 2 060 234 A1**
(43) Date of publication of application: **20.05.2009**
(21) Application number: 08253646.7
(22) Date of filing: 07.11.2008
(51) Int. Cl.: A61B 17/064

(54) **Staple with multiple cross sectional shapes**

(30) Priority: 14.11.2007 US 987812 P; 05.11.2008 US 264946
(71) Applicant: Tyco Healthcare Group, LP, North Haven, CT 06473 (US)
(72) Inventor: Soltz, Michael, New York (US); Broom, Jennifer (Oudemool), Branford, CT 06405 (US)
(74) Representative: Soames, Candida Jane

(57) **Abstract**

Surgical staples having at least two different cross-sectional shapes are useful for fastening and securing tissue. The staples disclosed have portions with different cross-sectional shapes.

## Description

### CROSS REFERENCE TO RELATED APPLICATIONS

This application claims benefit of U.S. Provisional Application Serial No. 60/987,812 filed November 14, 2007, the disclosures of which are hereby incorporated by reference herein, in their entirety.

### BACKGROUND

### 1. Technical Field

This application relates to a surgical staple. More particularly, this application relates to surgical staples having at least two cross-sectional shapes.

### 2. Background of Related Art

Surgical staples for fastening and securing tissue are known. The use of surgical staplers to apply staples to wound sites is also known. Several types of staplers, including endoscopic, laparoscopic, open, and circular staplers, may be used when applying staples to tissue.

Staples are available in various sizes and configurations. Disposable loading units containing surgical staples for use with surgical staplers are known. For example, when using an endoscopic linear stapler, the user may select a loading unit with staples of a specific size and arranged in one or more lines of staples, having a selected staple line length. Disposable loading units which accommodate the passage of a knife blade are also known. Staples having more than one cross-sectional shape are known. Cook, U.S. Patent No. 5,667,527, discloses a surgical staple with staple legs having weakened regions. Fasteners with a variety of shapes are known, e.g., U.S. Patent Application Publication No. 2002/0133181, U.S. Patent No. 2,323,362 to Weiss, and PCT Publication No. WO 2004/105621.

Surgical staples typically have a uniform cross-sectional geometry such as circular or rectangular. Although these cross-sectional geometries are used, improvements to the staple shape and geometry are desired.

### SUMMARY

In a first aspect of the present disclosure, a surgical staple includes a first leg, a second leg, and a backspan extending therebetween. The first leg and second leg each having a first portion adjacent to the backspan and a second portion. The first portion has a first cross-sectional shape and the second portion has a second cross-sectional shape which is substantially curvilinear. The first cross-sectional shape is different than the second cross-sectional shape. The second portion defines at least one angled surface. The at least one angled surface desirably defines a pyramid, a wedge, or a cone, and optionally forms a sharp tip or sharp edge. In certain embodiments, the first cross-sectional shape is polygonal, and in other embodiments square or rectangular. In certain preferred embodiments, the second cross-sectional shape comprises an oval or a circle.

In another embodiment, the staple includes a first leg, a second leg, and a backspan extending therebetween. The first leg and the second leg each have a first portion adjacent to the backspan and a second portion adjacent a penetrating end. The first portion has a first cross-sectional shape and the second portion has a second cross-sectional shape. The second cross-sectional shape is substantially curvilinear and the first cross-sectional shape is different than the second cross-sectional shape. The penetrating end forms a sharp tip. In certain embodiments, the sharp tip defines a shape that is a pyramid, wedge, cone or tetrahedron. The first cross-sectional may be an oval or circle. The second cross-sectional shape may be polygonal, square or rectangular. Additionally, the sharp tip desirably comprises a sharp edge, sharp point or apex.

### BRIEF DESCRIPTION OF DRAWINGS

Various preferred embodiments of the presently disclosed surgical stapling device are described herein with reference to the drawings, in which:
FIG. 1 is a perspective view of an endoscopic surgical stapling device;
FIG. 2 is a partial cross-sectional view of the endoscopic surgical stapling device of FIG. 1, showing the actuation shaft;
FIG. 3 is an exploded view of the cartridge and anvil portions of the endosopic surgical stapling device of FIG.1;
FIG. 4 is a partial perspective view of the anvil portion of the endoscopic surgical stapling device of FIG. 1;
FIG. 5 is a perspective view of a surgical staple in accordance with one embodiment of the present disclosure;
FIG. 6 is a perspective view of a surgical staple in accordance with another embodiment of the present disclosure;
FIGS. 7a-7h are various transverse cross-sectional shapes for a surgical staple in accordance with the present disclosure;
FIGS. 8a-8e are various transverse cross-sectional shapes for a tip of a surgical staple in accordance with the present disclosure.

### DETAILED DESCRIPTION OF EMBODIMENTS

Preferred embodiments of the presently disclosed surgical stapling device will now be described in detail with reference to the drawings in which like reference numerals designate identical or corresponding elements in each of the several views.

In the description that follows, the term "proximal," will refer to the end of the stapling device which is closest to the operator, while the term "distal" will refer to the end of the stapling device which is furthest from the operator.

The present disclosure is directed to a surgical staple. The surgical staple is deployed in tissue using a surgical stapling device. Surgical stapling devices envisioned with the present disclosure include but are not limited to open stapling devices, laparoscopic stapling devices, endoluminal stapling devices, and circular stapling devices. Examples of surgical stapling devices include U.S. Patent No. 7,044,353 to Mastri et al., U.S. Patent No. 6,953,139 to Milliman et al., U.S. Patent No. 6,644,532 to Green et al., and U.S. Patent No. 5,941,442 to Geiste et al., the disclosures of which are hereby incorporated by reference herein in their entirety. In the description that follows, the term "staple" includes but is not limited to staples, fasteners, and clips.

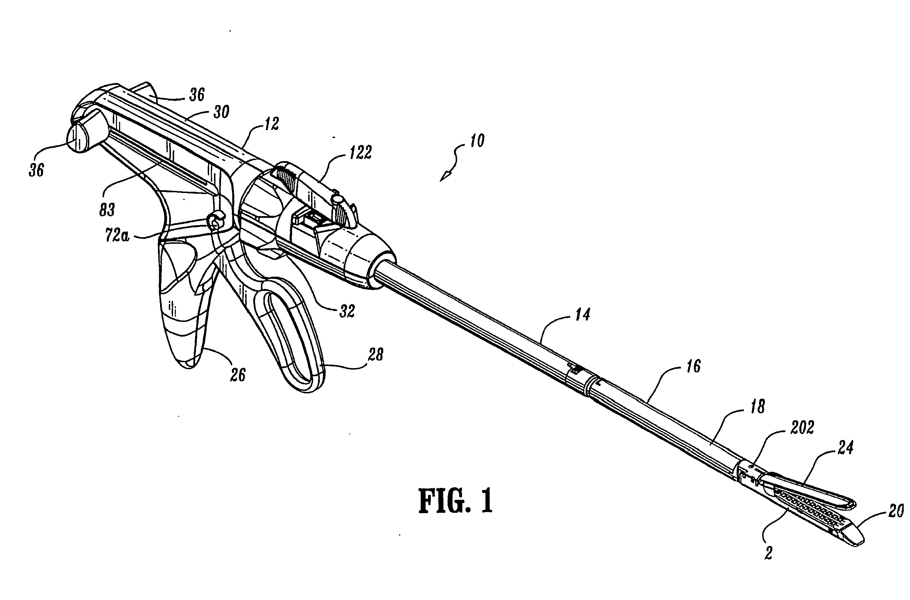

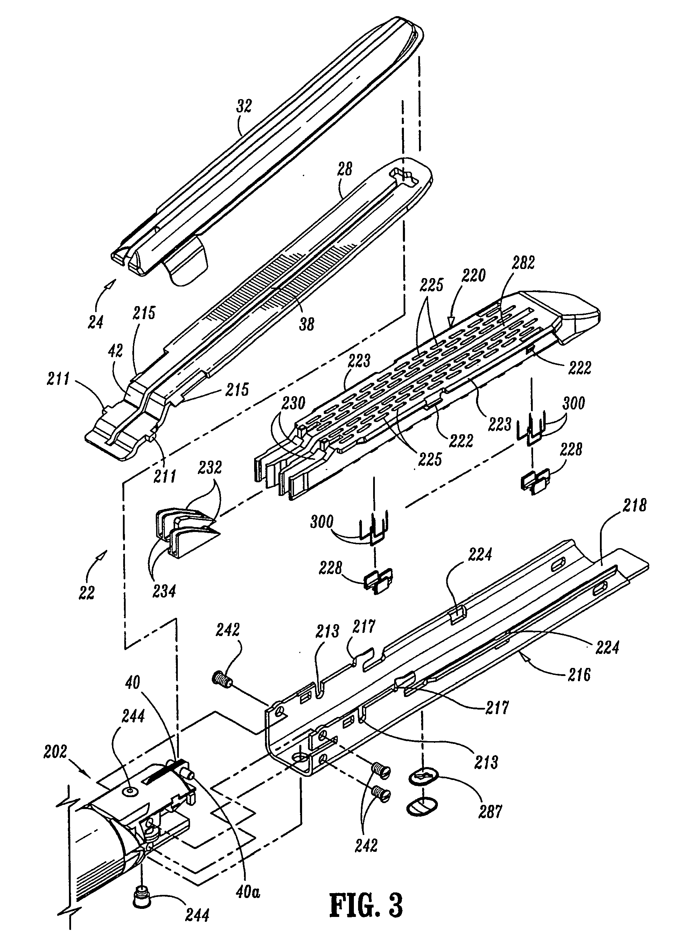

A surgical stapling device 10 which may be used to deploy a surgical staple in accordance with the present disclosure includes a handle assembly 12 and an elongated body 14. The elongated body defines a longitudinal axis. The length of elongated body 14 shown in FIG. 1 may vary to suit a particular surgical procedure. A single use loading unit, a,replaceable loading unit and/or disposable loading unit 16, such as a single use loading unit ("SULU"), is releasably secured to a distal end of elongated body 14. SULU 16 includes a proximal body portion 18, which forms an extension of elongated body 14, and a distal tool assembly 20 including a cartridge assembly 22 and an anvil assembly 24. In certain embodiments, the tool assembly 20 is pivotably connected to proximal body portion 18 about an axis substantially perpendicular to the longitudinal axis of elongated body 14. SULUs that do not include a pivotably connected tool assembly 20 may also be used. The tool assembly 20 includes a cartridge assembly 22, which houses a plurality of surgical staples 300. Anvil assembly 24 is movable in relation to cartridge assembly 22 between an open position spaced from cartridge assembly 22 and an approximated or clamped position in juxtaposed alignment with cartridge assembly 24. Staples are housed in the cartridge assembly 22 and applied as linear rows of staples having a preselected length, typically measuring from about 30 mm to about 60 mm, although other staple configurations and lengths are envisioned. In the embodiment shown, rows of staples are staggered with respect to each other. (See FIG. 3). Other staple configurations, patterns, and lengths are envisioned.

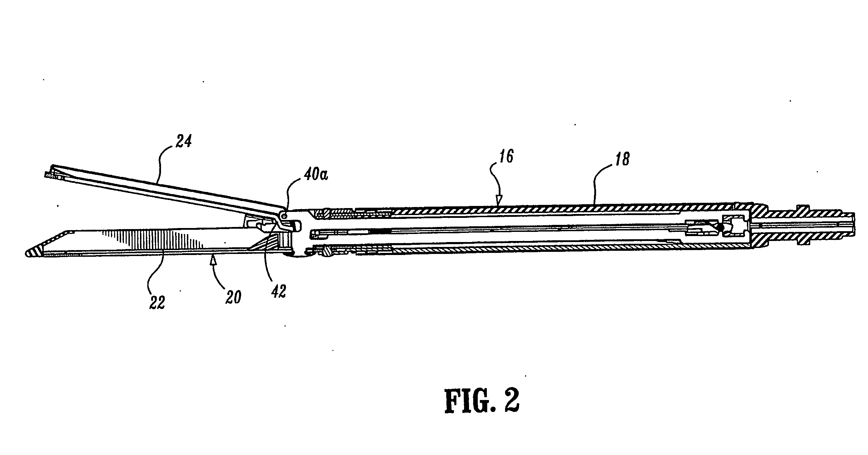

When stapling device 10 is employed, tissue is clamped between the cartridge assembly 22 and anvil assembly 24 through manipulation of a movable handle 28 by the operator. In manipulating the movable handle 28, the operator pivots the handle 28 toward a stationary handle member 26. The handle assembly 12 is arranged to distally advance a n actuation shaft (not shown) and/or rod, which engages a drive beam 266. As the actuation shaft is advanced, cam rollers 40a (see FIG. 2), located at the distal end of drive beam 266 engage a cam surface 42 on anvil assembly 24, clamping tissue between the anvil assembly 24 and cartridge assembly 22. The operator manipulates the handle 28 again to further drive the drive beam 266 and deploy surgical staples from the cartridge assembly 22 into tissue. As the drive beam 266 is driven distally, the drive beam 266 engages the actuation sled 234, guiding the actuation sled 234 through staple cartridge 22. The actuation sled 234 (FIG 3) advances pushers 228 through cartridge assembly 22 to sequentially eject staples 225 from slots 225 in the cartridge assembly 22, through tissue, towards the anvil assembly 24. The drive beam 266 includes a distally-facing knife 280, which severs tissue as the drive beam 266 moves distally. More than one actuation stroke may be required to fire all the staples from the loading unit 16 and cut tissue, depending on the relative size of the staple line for the particular DLU attached to the device.

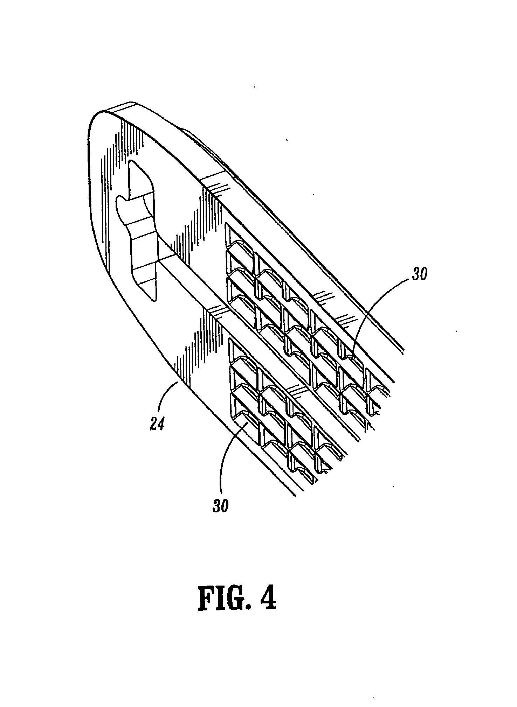

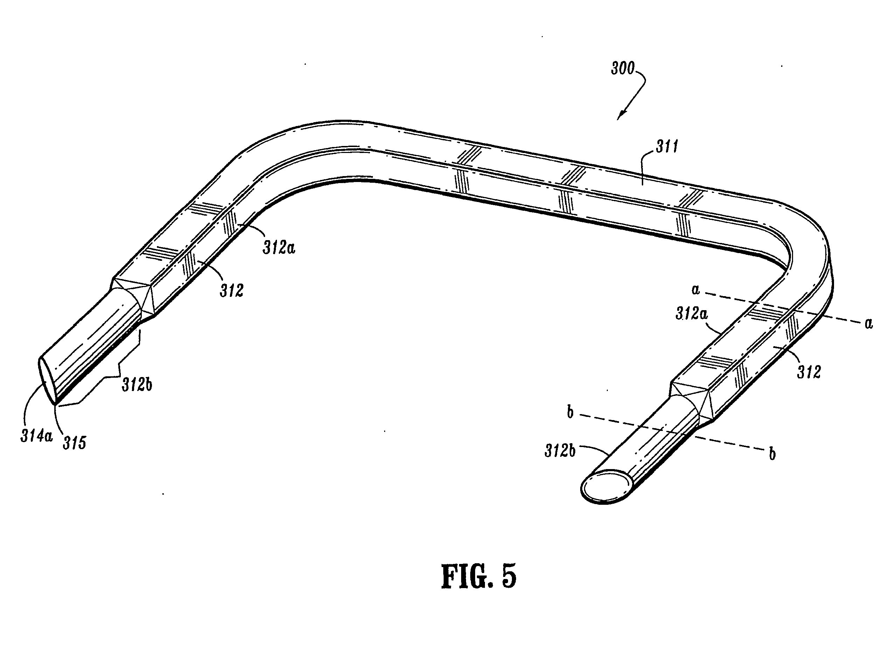

The actuation sled 234 includes cam wedges 232 that are arranged so that the pushers 228 translate vertically along cam wedges 232 as the actuation sled 234 travels distally. The pushers 228 translate within staple retention slots 225 and urge staples from retention slots 225 into staple deforming cavities 30 of anvil assembly 24. The staples are oriented in the retention slots 225 so that the ends of the staple legs 312 face the staple deforming cavities 30 and exit the retention slots 225 first. In the embodiment, the staple deforming cavities 30, which are shown in FIG. 4, are shaped to deform the staples generally into a "B" shape as the legs of the staples are driven against the anvil assembly 24. However, other formed staple shapes are contemplated. Once staples are ejected through slots 225, staple legs (FIG.5) then penetrate tissue and staple legs pass through tissue to engage the anvil assembly 24, where staples 300 form in staple deforming cavities 30 (FIG. 4). Staple legs then penetrate tissue a second time, completing the "B" shaped staple formation (not shown). In the present disclosure, other formed staple shapes are also contemplated.

Once staples are fired from the SULU 16, the SULU 16 can be removed from the device 10 and a new loading unit 16 may be assembled with the stapling device 10.

Surgical staples of the present disclosure include at least two legs. In one embodiment shown in FIG. 5, legs 312 are integral with a backspan 311, which extends therebetween. Each of the legs 312 includes a respective first portion, 312a, and a second penetrating portion, 312b. First portions 312a are connected to the backspan 311 and preferably integral therewith. The second, or penetrating portions 312b each terminate in a tip and define at least one angled surface 314a.

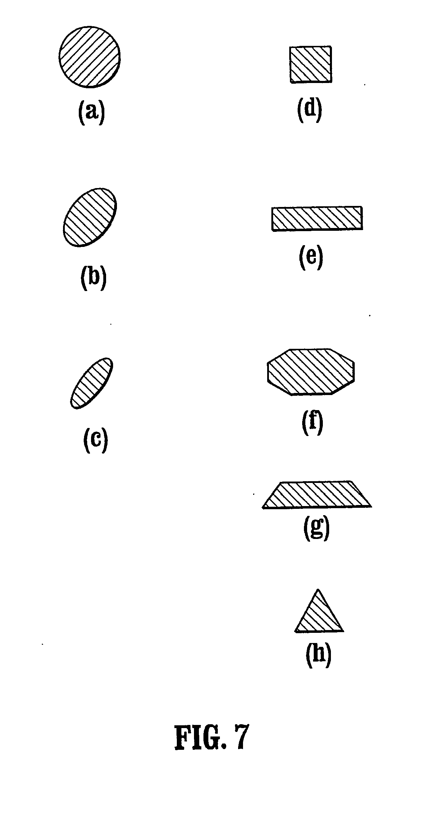

Staple legs 312 and backspans 311 may incorporate a variety of cross-sectional shapes as illustrated in FIGS. 7a through 7h. Furthermore, legs 312 need not be of the same length, at the same angle with respect to the backspan 311 or each other. Legs 312 can be disposed at different angles with respect to the backspan 311. Additionally, legs 312 can extend inwardly towards each other, or outwardly away from each other, or in different directions.

In a preferred embodiment, the staple legs 312 each have portions with different cross-sectional shapes. The first portion 312a of each of the legs 312 has a cross-sectional shape that is different from the second portion 312b of each of the legs 312. For example, each first portion 312a has a polygonal cross-sectional shape, whereas each second portion 312b has a curvilinear, which includes oval and circular, cross-sectional shape. (See FIG. 5). In another preferred embodiment, the first portion 312a has a rectangular or square cross-sectional shape, whereas the second portion 312b has a curvilinear cross-sectional shape. The rectangular or square cross-sectional shape generally twists less during staple formation compared to a curvilinear section. The penetrating portion 312b of the curvilinear-shaped section may eliminate undesirable edges and/or corners, for smoother penetration potentially resulting in decreased tissue damage.

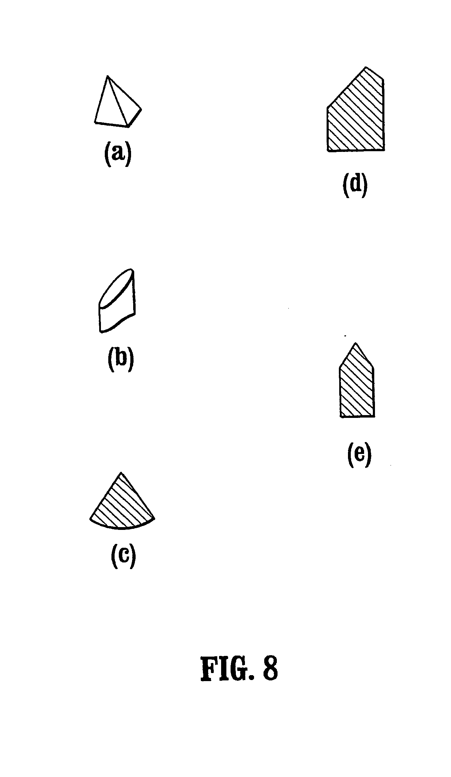

In further embodiments, the first portion 313 of the staple leg 312 has an oblong cross-sectional shape, including rectangular, oval and irregular shapes. The oblong cross-sectional shape is preferably oriented to encourage bending toward the backspan during formation. The second portion 312b has a curved cross-sectional shape, for example, oval or circular. The backspan 311 can be polygonal or other shapes such as square, oval, circular, rectangular and irregular shapes. Each of the legs 312 may have the same or different configuration as the other leg or legs 312. In certain embodiments, the backspan 311 of staple 300 defines a shape, which can be the same or different from the first portion 312a (FIGS. 7a-7h). For example, first portions 312a may define an oval shape (FIGS. 8a-8h), while backspan 311 may define a rectangular shape and second portions 312b may define a circular shape.

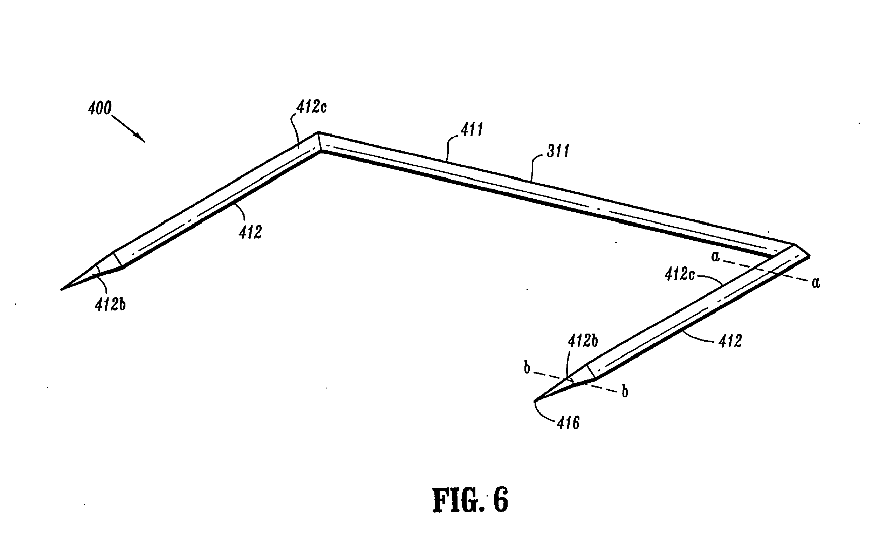

In another embodiment as shown in FIG. 6, staple legs 412 include a first portion 412a with a triangular cross-sectional shape while a second portion 412b has a curvilinear cross-sectional shape. Each of the legs 412 may have the same or different configuration as the other leg or legs 412. The backspan 411 defines a triangular cross-sectional shape while the tip on the second, penetrating portion 412b of staple leg 412 defines an apex 416.

As shown in FIGS. 8a-8e, angled surfaces 314a may define a variety of shapes, angles, and planes, and need not be of similar shape, size or length with respect to each other. The angled surface 314a of the penetrating portion 312b reduces the profile of the penetrating portion 312b, reducing the footprint of the staple 300 with respect to tissue penetration. The angled surface 314a may form a sharp tip 315 with a sharp edge (see FIG. 5), or may define an ' apex 416, as shown in FIG. 6. Furthermore, penetrating ends of staple legs 312b may have an angled surface including geometries such as pyramids, wedges, cones, tetrahedrons and the like. Additionally, sharp tips need not be of similar geometry with respect to each other.

Staples are comprised of materials within the purview of one skilled in the art including but not limited to metals such as titanium, steel, cobalt chromium, and magnesium and the like. Staples may also be derived from polymers including polylactides, polyglycolides, polycaprolactones, polyhydroxybuterates, hydroxyalkoanates, and combinations thereof. Staples may also be comprised of shape memory polymers and shape memory such as nitinol. Staples may be absorbable, non-absorbable and combinations thereof.

Staples including various cross-sectional shapes can be made using techniques within the purview of those skilled in the art, including but not limiting to extruding, drawing, injection molding, machining, grinding, cold rolling and work hardening.

It will be understood that various modifications may be made to the embodiments disclosed herein. For example, the above described staple may be incorporated into a variety of surgical instruments which include loading units and is not limited to use on endoscopic, open and circular staplers. Therefore, the above description should not be construed as limiting, but merely as exemplifications of various embodiments. Those skilled in the art will envision other modifications within the scope and spirit of the claims appended hereto.

Surgical staples having at least two different cross-sectional shapes are useful for fastening and securing tissue. The staples disclosed have portions with different cross-sectional shapes.

## Claims

1. A staple comprising:
a first leg, a second leg, and a backspan extending therebetween;
the first leg and the second leg each having:
a first portion adjacent to the backspan and a second portion, wherein the first portion has a first cross-sectional shape and the second portion has a second cross-sectional shape;
the second cross-sectional shape being substantially curvilinear and the first cross-sectional shape being different than the second cross-sectional shape; and,
the second portion defining at least one angled surface.

2. The staple according to claim 1, wherein the second cross-sectional shape comprises an oval.

3. The staple according to claim 1, wherein the second cross-sectional shape comprises a circle.

4. The staple according to claim 1, wherein the first cross-sectional shape is polygonal.

5. The staple according to claim 4, wherein the first cross-sectional shape is square.

6. The staple according to claim 4, wherein the first cross-sectional shape is rectangular.

7. The staple according to claim 1, wherein the at least one angled surface forms a sharp edge.

8. The staple according to claim 1, wherein the at least one angled surface defines a pyramid.

9. The staple according to claim 1, wherein the at least one angled surface defines a wedge.

10. The staple according to claim 1, wherein the at least one angled surface defines a cone.

11. A staple comprising:
a first leg, a second leg, and a backspan extending therebetween;
the first leg and the second leg each having:
a first portion adjacent to the backspan and a second portion adjacent a penetrating end, wherein the first portion has a first cross-sectional shape and the second portion has a second cross-sectional shape;
the second cross-sectional shape being substantially curvilinear and the first cross-sectional shape being different than the second cross-sectional shape; and
the penetrating end forms and a sharp tip.

12. The staple according to claim 11, wherein the sharp tip defines a shape selected from the group consisting of a pyramid, wedge, cone, and tetrahedron; or wherein the first cross-sectional shape comprises an oval; or wherein the first cross-sectional shape comprises a circle.

13. The staple according to claim 11, wherein the second cross-sectional shape is polygonal.

14. The staple according to claim 13, wherein the second cross-sectional shape is square; or wherein the second cross-sectional shape is rectangular.

15. The staple according to claim 11, wherein the sharp tip comprises a sharp edge; or wherein the sharp tip comprises a sharp point; or wherein the sharp tip defines an apex.
